# EUROPEAN PATENT APPLICATION

(11) **EP 4 015 046 A1**
(43) Date of publication of application: **22.06.2022**
(21) Application number: 21215404.1
(22) Date of filing: 17.12.2021
(51) Int. Cl.: A61Q 17/04, A61K 8/37, A61K 8/41, A61K 8/44, A61K 8/49

(54) **SUN PROTECTION TOPICAL COMPOSITIONS**

(30) Priority: 17.12.2020 LU 102317
(71) Applicant: Société De Recherche Cosmétique S.à.r.L., 2314 Luxembourg (LU)
(72) Inventor: NOUAL, Amandine, 1017 VG Amsterdam (NL); DELAMARE, Sarah, 78000 Versailles (FR)
(74) Representative: Lecomte & Partners

(57) **Abstract**

The invention concerns a homogeneous sun protective topical composition comprising: an effective amount of a UV filter system comprising a mixture of following compounds: Diethylamino Hydroxybenzoyl Hexyl Benzoate (DHHB), Ethylhexyl Triazone (EHT), and Bis-ethylhexyloxyphenol Methoxyphenyl Triazine (BEMT); and a mixture of ester compounds comprising isopropyl lauroyl sarcosinate, and diisopropyl sebacate, said ester compounds mixture being in quantity just required for producing said homogeneous sun protective topical composition, achieving a SPF value of at least 10. The invention also relates to a ready to use sunscreen preparation comprising the homogeneous sun protective topical composition.

## Description

The invention is directed to the field of cosmetics, and more particularly to sun protection topical compositions with a high Sun Protection Factor (SPF).

It is well known that ultraviolet (UV) radiations, in particular UVA and UVB, are harmful to human skin and cause different types of skin damages. Indeed, UVB radiations (wavelength between 290 to about 320 nm) are responsible for sunburns and account for some skin cancers. Upon these radiations, epidermal biomechanical properties undergo long term detrimental changes, leading to premature ageing of the skin. For a long time considered as less harmful than UVB, it has been shown that UVA radiations (wavelength between 320 nm and 400 nm) while producing tanning of the skin, contribute also to sunburns. Moreover, these UVA radiations, under quite normal everyday conditions, are also sufficient to damage over time the collagen and elastin fibres, which are important elements for the structure and strength of the skin.

Therefore, to protect the human skin against sunlight and particularly against UVA and UVB radiations, it is thus desirable that the sun protection products, which are applied directly on the skin, provide UV filtering over the range of UVA and UVB.

A range of UV filters against UVA, UVB or both which can be used in cosmetic sun protection compositions have been developed and are available for the cosmetic industry. In most countries, these UV filters are in particular summarized in the form of approved lists, such as Annex VI of the European Cosmetic Regulation n°1223/2009 which authorizes 31 UV filters.

Typically, two types of UV filters enable skin photo-protection against UVA and/or UVB radiations, namely organic filters and mineral filters. Organic filters, also called chemical filters, absorb UV radiations instead of the skin. Some of these organic filters are liquid and the others are under a solid form (i.e. in powder form) but they are mainly liposoluble. In contrast, inorganic UV filters which are also called physical filters, reflect the UV radiations, and they are inert and opaque powders.

Commonly, the protection of sun products against UVB radiations is reflected by the sun protection factor (SPF). This factor indicates the protection that the human skin gets from exposure to these radiations, and it is the foremost criteria watched and demanded by the consumers, in parallel with getting sunscreen products which are not heavy, oily, and/or give a sticky sensation on the skin.

One of the ways of achieving a high protection against sun's UV, is to incorporate high levels of UVA, UVB and broad spectrum filters in the sun protection products. Organic filters achieve protection mainly by absorbing the UV radiations whereas inorganic filters mainly reflect the UV radiations. Under certain circumstances, inorganic filters often involving nanoparticles might not be desirable for the consumers and the environment. However, effective known organic UV filters, particularly solid lipophilic organic UV filters, when they are used in high concentration to prepare sunscreen products, present a problem of solubility and moreover lead to unstable sunscreen products since these filters tend to (re)crystallise over time. This can occur in different galenic forms of the sun protection products/compositions. A consequence of this (re)crystallisation is that the UV protection of the sunscreen products is significantly decreased.

Moreover, lipophilic organic UV filters, when they are used in a sun topical protection product, conduct to sun products presenting a heavy, oily and sticky sensation leading to a discomfort when applied on the skin. Problems of solubility and (re)crystallisation of high concentration of solid lipophilic organic UV filters can provide sun protection compositions changing in color, visual, sensory and physicochemical aspects, having heterogeneous formula and impacting the quality of the film leaved on skin. This actually tends to decrease the efficiency of the spreading of these compositions on the skin leading to a poor UV protection.

To solve the problems of solubility of solid organic UV filters, and more particularly lipophilic organic filters, the liquid organic UVB filter ethylhexyl-2-cyano-3,3-diphenylacrylate (INCI: octocrylene) has been used for a long time by cosmetic formulators as emollient ester/solvent and also as photo-stabilizer. Similarly, the (lipophilic) organic UV filters, ethyhexyl methoxycinnamate, homosalate and ethylhexylsalicylate are also widely used to dissolve and stabilize solid lipophilic organic UVA and UVB filters, respectively, in addition to their function of UV absorber. However, these four UV filters are controversial for being suspected to have a negative impact on human health and the environment.

Concerning the undesired sensorial properties of sun topical protection products containing lipophilic organic UV filters, this problem is most commonly solved by the addition of siloxanes and or derivatives, such as dimethicone, which are well known in the cosmetic industry for their high sensorial properties. They are particularly used to reduce the sticky and greasy feeling on the skin. However, siloxanes and derivatives are currently being avoided due to their ecological footprint and their potential occlusive effect on the skin.

The invention has for technical problem to overcome at least one of the drawbacks of the prior art. More specifically, the invention has for technical problem to provide stable and homogeneous sun topical protection compositions over time with a high SPF, preferably associated with a light sensation on the skin, a non-greasy sensory effect, an easy and/or pleasant spreadability and optimized naturalness. The main difficulty is linked to the selection of solid organic filters mixture, wherein each filter compounds has its own solubility depending on the nature and quantity of solubilizing ester, and ester compounds allowing to the filters mixture to be fully solubilized therein while in the same time providing naturalness and sun protection.

According to one aspect, the invention relates to a homogeneous sun protective topical composition comprising:
1) an effective amount of a UV filter system comprising a mixture of following compounds:
   a) Diethylamino Hydroxybenzoyl Hexyl Benzoate (DHHB),
   b) Ethylhexyl Triazone (EHT), and
   c) Bis-ethylhexyloxyphenol Methoxyphenyl Triazine (BEMT); and
2) a mixture of ester compounds comprising:
   a) Isopropyl lauroyl sarcosinate, and
   b) diisopropyl sebacate,
said ester compounds mixture being in quantity just required for producing said homogeneous sun protective topical composition.

The Applicant has shown that the homogeneous sun protection topical composition which comprises a UV filter system with specific ester compounds exhibits several advantages. Said composition is a good compromise between a high Sun Protection Factor (SPF) of at least 10, preferably of at least 30, more preferably 50 and above, entire solubility of the specific UV filter system based on solid organic compounds, thus excluding liquid organic filters, in a minimal quantity of the mixture of said ester compounds, for optimizing the viscosity of the composition, the cosmetic naturalness, and the stability over time for several months or, even better, for several years. In some applications, the homogeneous sun protection topical composition is devoid or substantially free of compounds selected from the group consisting of siloxanes and derivatives thereof, such as dimethicone; octocrylene; ethyhexyl methoxycinnamate; homosalate; ethylhexylsalicylate and the like, and mixtures thereof. In the context of the invention, "substantially free" means that these compounds may be present in an amount as low as 2 wt% maximum, even better less than 1 wt%.

More specifically, the obtained sun protective topical composition, otherwise named "composition", includes a specific mixture of ester compounds, isopropyl lauroyl sarcosinate and diisopropyl sebacate, used in a minimal quantity for the dissolution of a mixture including Diethylamino Hydroxybenzoyl Hexyl Benzoate (DHHB), Ethylhexyl Triazone (EHT), and Bis-ethylhexyloxyphenol Methoxyphenyl Triazine (BEMT) present in an effective amount, for obtaining a SPF, which is based on the UV filter system, of at least 10, better of at least 30, especially of from 10 to 75, preferably of from 30 to 75, better of from 35 to 75, more preferably of from 37 to 72. Accordingly, said composition is a good compromise between the specific choice of ester compounds with a minimal quantity thereof, advantageously selected for their naturalness, for dissolution of specific UV filter solid organic compounds, and providing a SPF value of at least 30. It is the merit of the invention since the Applicant has found how to provide the composition including the three filters in an effective amount with also specific esters generally associated with desired amounts.

The mixture of ester compounds of the invention is selected to provide an enhanced naturalness to the composition and, as already mentioned, to allow solubility of the UV filter system. The naturalness may be increased by more than twice fold that of known ester compounds in a ready to use sunscreen final preparation.

According to the invention, the effective amount of the UV filter system is required for obtaining, with a minimal number of solid organic compounds, i.e. DHHB, EHT and BEMT, the desired SPF, complete solubility in a specific ester mixture and compliance with various regulations regarding maximum allowed quantity of UV filter organic compounds in ready to use sun protection topical preparations. DHHB was selected for its property regarding efficient filtering action of UVA, EHT for efficient filtering action of UVB and BEMT for its efficient filtering action of UVA + UVB.

In said UV filter system, DHHB may preferably be the main compound in terms of relative weight%. DHHB may be efficiently solubilized in said mixture of ester compounds and advantageously provides good protection against UVA. EHT may be present in quantity complying with various country regulations and that may be solubilized in the mixture of esters. BEMT quantity is generally less than DHHB and/or EHT quantity or weight% for also complying with the maximum weight or relative weight% authorized by various country regulations. In some preferred embodiments, the BEMT quantity may be of from 30% to 60% less than the maximal accepted quantity, according to some countries regulations, either in a homogeneous sun protective topical composition or in a ready to use sunscreen preparation comprising said homogeneous sun protective topical composition.

In the context of the invention, the relative weight% means the % weight of any compound based on 100 wt% of the considered system in which they are dissolved or present. As an example, for 100 g of the total weight of the considered system, there may be x relative wt% of A, y relative wt% of B, z relative wt% of C etc., the total being 100 g or 100 wt%.

In some more preferred embodiments, the UV filter system is the mixture wherein, independently each other, the relative weight%, based on 100% weight of the UV filter system, for DHHB is of from 35 to 60, for EHT is of from 10 to 35 and for BEMT of from 5 to 30. More preferably, independently each other, DHHB relative weight% is of from 40 to 60, EHT relative weight% is of from 15 to 30, and BEMT relative weight% is of from 7 to 27. Advantageously, the weight% ratio between the UV filter system and the mixture of ester compounds may be of from 0,20 to 1,5 or even in some alternate embodiments of from 0,1 to 1,5. In some applications of the composition, for example in ready to use sunscreen preparations, like sprays, creams, lotions, foams, gels, milks and the like, the invention allows to have a relative weight% that is of from 0,30 to 1,4 providing a composition which is the less oily and viscous. In some other embodiments, where a ready to use oil preparation including said composition is prepared, the weight% ratio may be of from 0,22 to 0,5.

The UV filter system may include at least one or some additional solid organic filter(s).

The UV filter system may include an amount of from 0 to 25 relative wt% (based on 100 wt% of the UV filter system) of phenyl benzimidazole sulfonic acid (PBSA). In some aspects, the PBSA is omitted (0 wt%). The presence of PBSA, preferably in an amount of relative wt% of 12 to 25, may advantageously increase the naturalness of the ready to use sunscreen final preparation related to emulsion ones, like sprays, creams, lotions, foams, gels, milks and the like, while, in some applications, lowering the amount of EHT, for example 1,5-2 fold.

The mixture of ester compounds comprises isopropyl lauroyl sarcosinate in a range of relative wt% preferably of from 2 to 50, or even of from 5 to 50, more preferably from 6 to 50, even better, of from 7 to 45. When the composition of the invention is included in oils, the wt% of isopropyl lauroyl sarcosinate may be lowered, in some embodiments, to the minimal quantity of from 5 to 20, more preferably of from 6 to 15, even better of from 7 to 10. In fact, due to the presence of other oily compounds, said quantity is as low as possible. For other uses, as sprays, creams or lotions, milks and the like, the wt% relative ratio of isopropyl lauroyl sarcosinate may be of from 10 to 50 more preferably of from 12 to 45. In some alternative embodiments, the relative wt% may be as low as 1-10.

The mixture of ester compounds also comprises diisopropyl sebacate.

The diisopropyl sebacate may preferably be present in a relative wt% of from 5 to 65, more preferably of from 10 to 60, irrespective of the kind of the final sunscreen preparation, i.e oil, cream, lotion, spray, gels, milks and the like.

One of the advantages of the invention is the specific use or selection of both diisopropyl sebacate and isopropyl lauroyl sarcosinate which are acting in synergy for the sought technical effects, for example, enhanced naturalness, for example of 30%-100%, preferably 50%-80%, more preferably, 50%-70%, solubility of the UV filter system, especially with the relative wt% mentioned above. Another aspect, is not only the use of both diisopropyl sebacate and isopropyl lauroyl sarcosinate as such, but the synergic combination thereof with the specific UV filter system.

The mixture of ester compounds may advantageously comprise of from 0 to 70 relative wt% of dibutyl adipate. The presence of the additional dibutyl adipate in the mixture of esters results in optimized solubilization of the three compounds of the UV filter system, with regard to their respective allowed amounts. When the composition of the invention is included in oils, the relative wt% of dibutyl adipate may preferably be within a range of from 8 to 18, and, due to the presence of other oily compounds, said quantity is as low as possible, whereas in sprays, creams, gels, lotions, milks and the like, the relative wt% of dibutyl adipate is preferably within a range of from 15 to 70, more preferably of from 18 to 65. In some alternative embodiments, dibutyl adipate is omitted or may be present in an amount as low as 0,1 to 10, for example, in oily ready to use sunscreen preparations.

In some alternate embodiments, BEMT may also be present in a liquid mixture further comprising at least acrylates/C₁₂₋₂₂ alkyl methacrylate copolymers and water. In such a case, the liquid mixture including BEMT cannot be used in oil sunscreen preparation. Such liquid mixture is preferred due to its naturalness, even if it slightly impairs the sensory of the ready to use sunscreen preparation without being however critical for its use.

The mixture of ester compounds may comprise at least one further ester with the proviso that the provided naturalness is not altered, even whether the presence thereof if not mandatory.

The at least one further ester may be selected from the group consisting in, based on 100 wt% of the esters mixture, of from 0 to 35 relative wt%, more preferably of from 0 to 30 relative wt% of C₁₂-C₁₅ alkylbenzoate, of from 0 to 60, more preferably from 0 to 55 relative wt% of coco caprylate/caprate, of from 0 to 10, more preferably of from 0 to 5 relative wt% of diethylhexyl succinate, of from 0 to 20, more preferably of from 15 to 20 relative wt% of isoamyl laurate and of from 0 to 10, more preferably of from 0 to 5 relative wt% of dextrin myristate. At least one among these esters may be used for an oil final sunscreen preparation. Such compounds advantageously allow the presence of a minimal amount of an oil phase, different from the esters mixture. Such additional ester compounds provide an enhanced solubility of the UV filter system. Specifically, dextrin myristate may advantageously be incorporated into the composition due to its film-forming properties improving by more than 30% the sun protection effect. The above esters list is not limitative.

According to an alternate embodiment, the UV filter system is only consisting in DHHB, EHT and BEMT, providing the desired technical effect(s).

The invention also relates to a ready to use sunscreen preparation comprising the homogeneous sun protective topical composition. The homogeneous sun protective topical composition is a base or an intermediate homogenous sun protective composition produced to be used for preparing a ready to use sunscreen preparation for direct topical uses. Said sunscreen preparation is classically including some other excipients usually found in said preparations in the form of oil, spray, cream, lotion, milk, gel, foams, bi-phases (constituting the phase), and the like. These ready to use sunscreen preparations are produced by means known to the one skilled in the art.

Typically, the homogeneous sun protective topical composition content, expressed in wt%, vs ready to use sunscreen preparation, may be of from 25 wt% to 95 wt%, more preferably of from 25 wt% to 85 wt% (based of 100 wt% of said preparation). When the ready to use sunscreen preparation includes oil phase, the composition content may then be the highest, for example of from 70 wt% to 85 wt%.

In some further embodiments, the ready to use sunscreen preparation may include, based on 100 wt% of said preparation, of from 8 to 10 wt% of DHHB, of from 2 wt% to 5 wt% of EHT, of from 1 wt% to 6 wt%, more preferably of from 1,5 to 5 wt% of BEMT, of from 3 wt% to 10 wt% of isopropyl lauroyl sarcosinate and of from 2 wt% to 11 wt% of diisopropyl sebacate. Said wt% of each compound of the invention may be considered independently each other.

Said ready to use sunscreen preparation may further include, depending upon use, independently each other, of from 6 wt% to 10 wt% of dibutyl adipate, of from 5 w% to 7 wt% of C₁₂-C₁₅ alkylbenzoate, of from 30 wt% to 40 wt% of coco caprylate/caprate, of from 8 wt% to 12 wt% of isoamyl laurate, of from 1 wt% to 4 wt% of phenyl benzimidazole sulfonic acid (PBSA) and of from 1 wt% to 3 wt% of dextrin myristate, or mixtures thereof.

As mentioned above, the ready to use sunscreen preparation may classically contain various non limitative excipients selected from the group consisting of, water, base oil, oily products, preservatives, colorant, fragrances, actives ingredients, pH adjustor(s), organic acids and vitamins, and mixtures thereof, in some classical contents, with the proviso that they do not impair the technical effects of the invention.

The whole ingredients contents, containing the UV filter system and the two esters, is based on 100 wt% of the ready to use sunscreen preparation.

The following experimental section and examples will more detail the preferred embodiments within the scope of the invention, without limiting its scope.

### Experimental section

The SPF data of the sun protection ready to use sunscreen preparations have been determined *in silico* with the BASF sunscreen simulator (https://www.sunscreensimulator.basf.com).

The measurements of SPF *in vivo* were carried out according to ISO 24444 :2010.

The following examples illustrate some ready to use sunscreen preparations including the homogeneous sun protective topical composition. All solid organic compounds constituting the UV filter system were totally dissolved in the mixture of ester compounds and in the phase according to classical implementation protocols and using means known to the one skilled in the art. In the following examples, when appropriate, the total wt% of compounds of the preparations are 100 wt%, meaning that the given wt% of each compound is selected so that the sum of all compounds complies with this requirement.

### Example 1

This example shows the full content of a ready to use sunscreen preparation (Table 1) including the homogeneous sun protective topical composition (UV filter system and mixture of ester compounds) and the phases for facial and body topical spray.

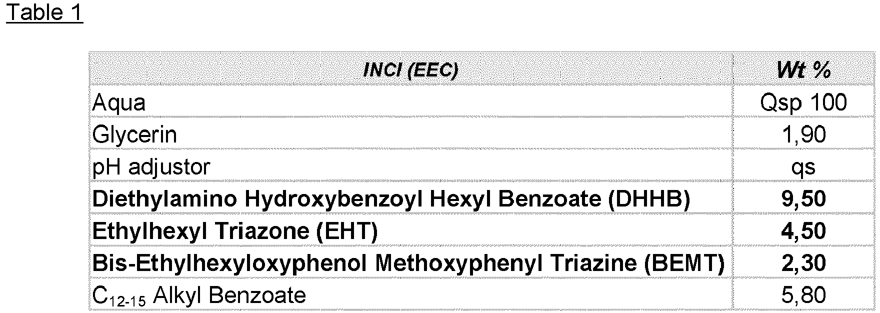

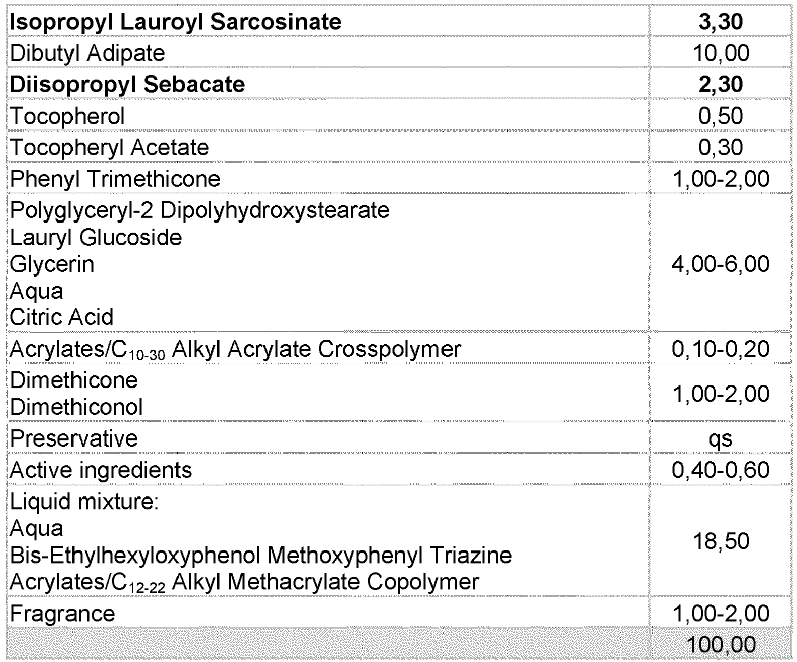

In bold : essential compounds of the invention
Calculated SPF: 52,3
*In vivo* SPF: 64,5

### Manufacturing procedure:

Heat water to 80°C under stirring;
Add aqueous components;
Keep the temperature of the stirred mix to 80°C.

At the same time, mix the organic UV filters and esters while heating up to 80°C until fully homogeneous;
Add the other oily compounds while keeping up to 80°C;

Add the oily phase to the aqueous phase while stirring and keeping at 80°C.

Homogenize under vigorous stirring during 15 minutes.

After this 15 minutes of emulsification, let the mix cool down under normal agitation.

At room temperature, add the last components (preservatives, active ingredients and fragrance).

Active ingredients are those classically used in the field of the invention, such as plant extracts (flowers, fruits and the like), which are added at room temperature. The preparation is stable, i.e. 6 months at 4°C, 25°C, and 3 months at 40°C.

From Table 1, it can be deduced that the DHHB wt% content is less than 10 wt%, the latter value being the maximal content allowed by several country regulations, like EEC, China, Australia, Korea, Asian countries (Indonesia, Thailand, Vietnam, Philippines etc.). Same applies for EHT, which maximal content allowed is 5 wt%, and BEMT, which maximal content allowed is 10 wt%. BEMT is also contained in the liquid mixture (18,5 wt%).

The facial and topical spray of this example also includes C₁₂₋₁₅ Alkyl Benzoate as an ester compound.

The SPF *in vivo* is high, providing desired high performances of such a preparation.

### Example 2

This example 2 shows the full content of a ready to use sunscreen preparations (Table 2) including the homogeneous sun protective topical composition (UV filter system and mixture of ester compounds) and the phase for facial and body topical cream.

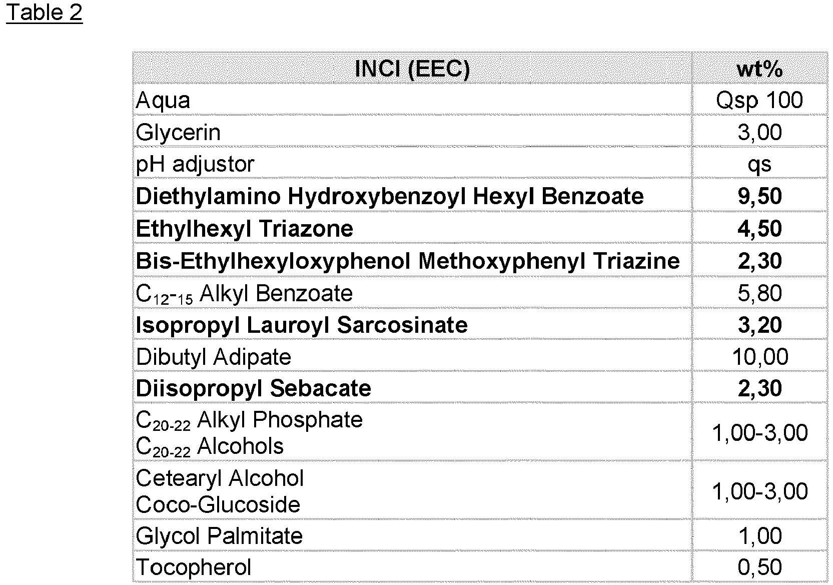

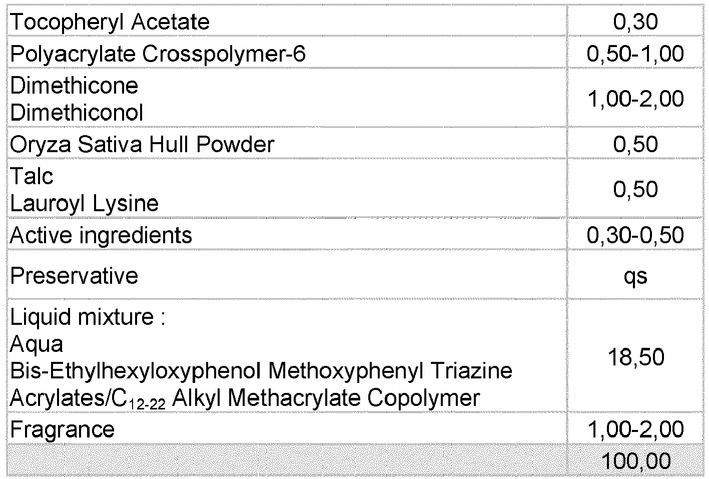

In bold : essential compounds of the invention
Calculated SPF: 52,3
*In vivo* SPF: 70,4

### Manufacturing procedure:

Same to example 1.

The preparation is stable, i.e. 6 months at 4°C, 25°C, and 3 months at 40°C. Same remarks as for example 1 do apply here.

The SPF *in vivo* is high, providing desired high performances of such a preparation.

### Example 3

This example 3 shows the full content of a ready to use sunscreen preparation (Table 3) including the homogeneous sun protective topical composition (UV filter system and mixture of ester compounds) and the phase for facial and body milk.

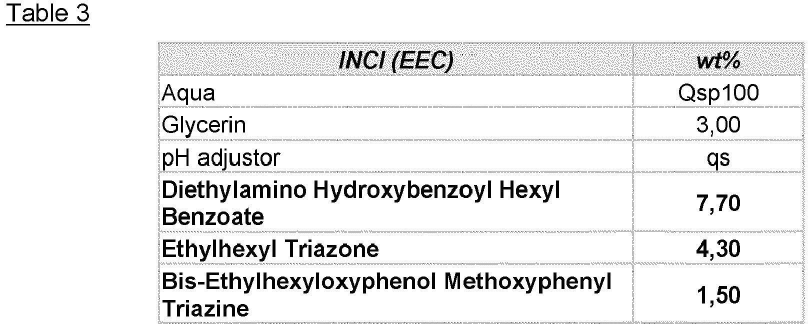

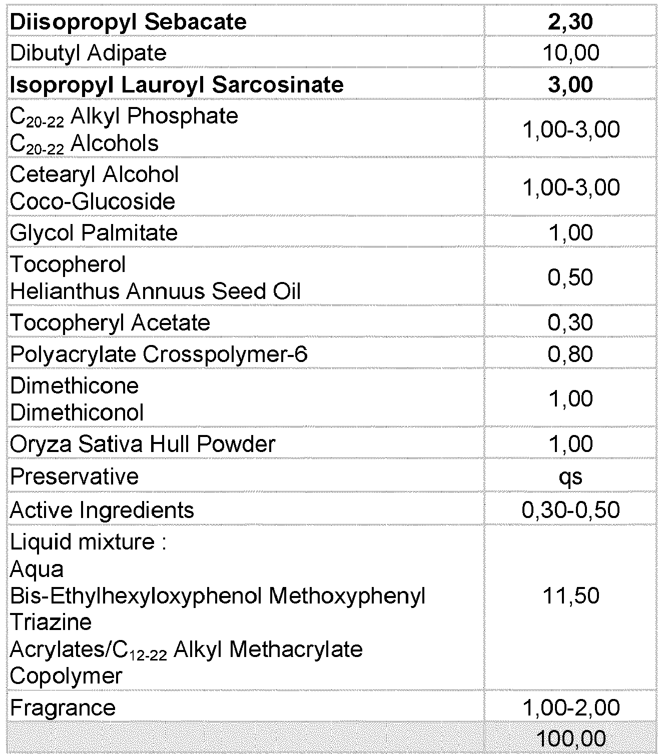

In bold : essential compounds of the invention
Calculated SPF: 39,9
*In vivo* SPF: 51,5

### Manufacturing procedure:

Same to example 1.

The preparation is stable, i.e. 6 months at 4°C, 25°C, and 3 months at 40°C.

Same remarks as for example 1 do apply here. BEMT is also contained in the liquid mixture (11,5 wt%).

### Example 4

The example 4 shows all compounds in a ready to use sunscreen preparation (Table 4) including the homogeneous sun protective topical composition (UV filter system and mixture of ester compounds) and an oil phase.

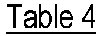

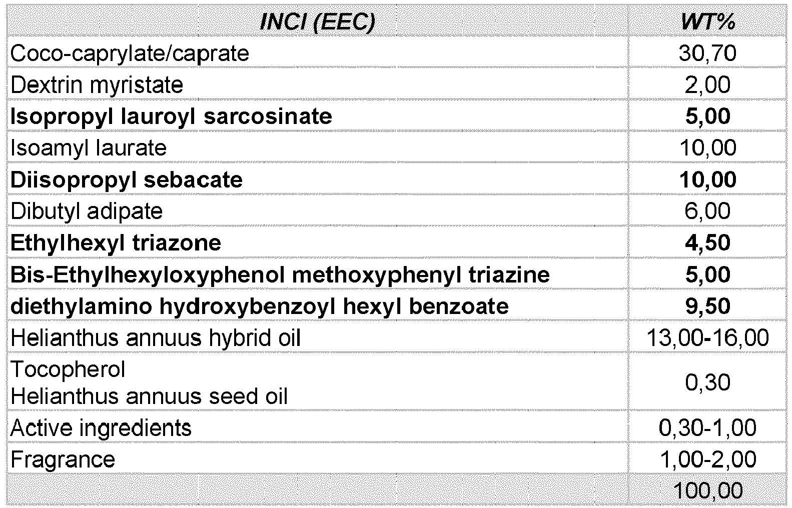

In bold : essential compounds of the invention
Calculated SPF: 41,4
*In vivo* SPF: 34,7

### Manufacturing procedure :

Under moderate stir, heat esters up to 85°C until fully homogeneous.

Add the organic UV filters and stir under normal agitation until completely homogeneous while maintaining the temperature at 75°C.

Cool down until room temperature while stirring.

Add the active ingredient and fragrance below 30°C.

The oil preparation is stable, i.e. 6 months at 4°C, 25°C, and 3 months at 40°C, and there is no crystallisation.

This ready to use oil preparation includes apart the esters mixture of the invention, other esters selected from coco caprylate/caprate in a high amount of 30,7 wt%, isoamyl laurate and dextrin myristate.

### Example 5

This example 5 shows the full content of a ready to use sunscreen preparations (Table 5) including the homogeneous sun protective topical composition (UV filter system and mixture of ester compounds) and the phase for milk and cream use.

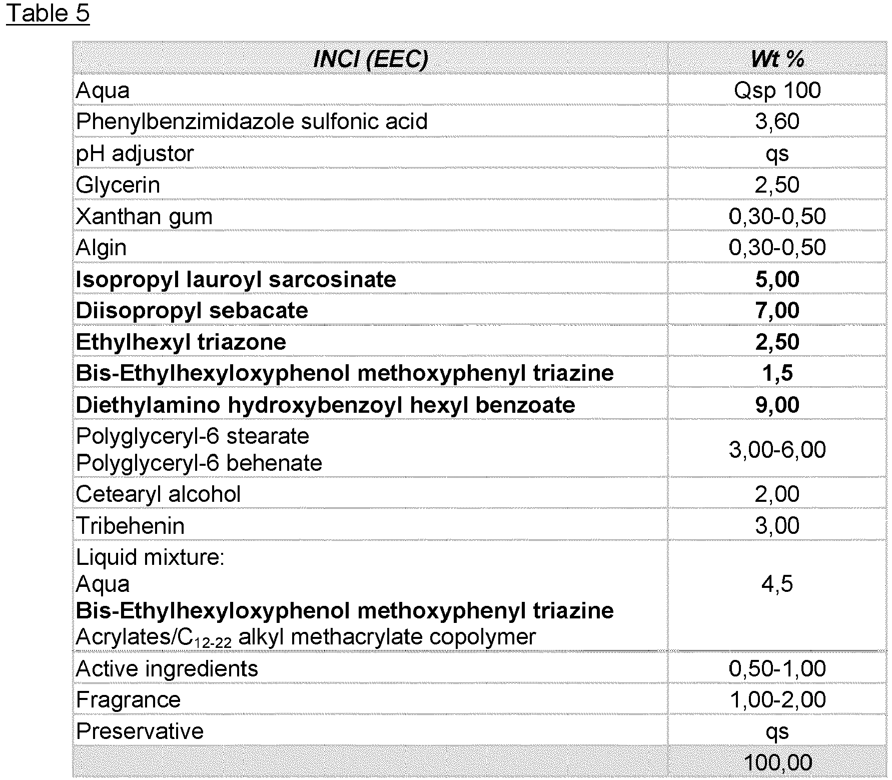

In bold : essential compounds of the invention
Calculated SPF: 41,5

### Manufacturing procedure:

Similar to example 1.

The preparation is stable at least one month at 4°C, 25°C, 40°C and 50°C.

This ready to use sunscreen preparation does not include dibutyl adipate as ester compound. The UV filter system comprises an additional filter as phenyl benzimidazole sulfonic acid which allows to lower the amount of EHT by about 2 times, compared to other ready to use preparations of examples 1-3. BEMT filter is here contained in the liquid mixture (6 wt%).

### Example 6

This example 6 depicts a ready to use sunscreen preparation (Table 6) including the homogeneous sun protective topical composition (UV filter system and mixture of ester compounds) and the phase for milk and cream use.

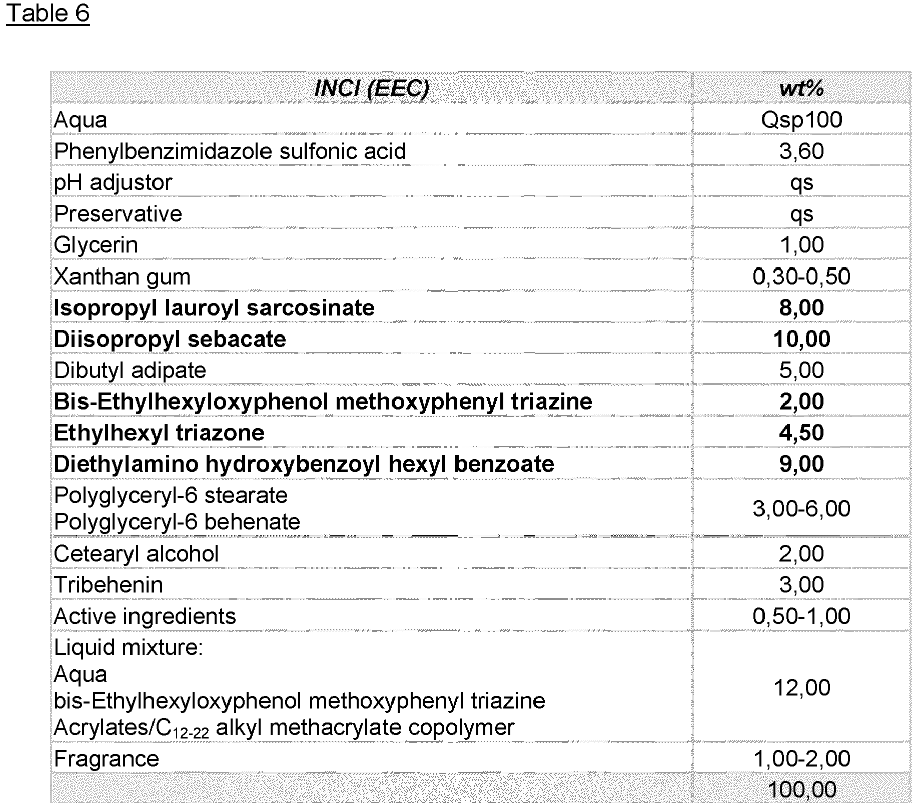

In bold : essential compounds of the invention
Calculated SPF: 65,7

### Manufacturing procedure:

Similar to example 1.

The stability of the preparation is of at least 1 month at ambient temperature.

Same remarks as for example 1 do apply here. BEMT is also contained in the liquid mixture (12 wt%).

### Example 7

The example 7 shows all compounds in a ready to use sunscreen preparation (Table 7) including the homogenous sun protective topical composition (UV filter system and mixture of ester compounds) and an oil phase.

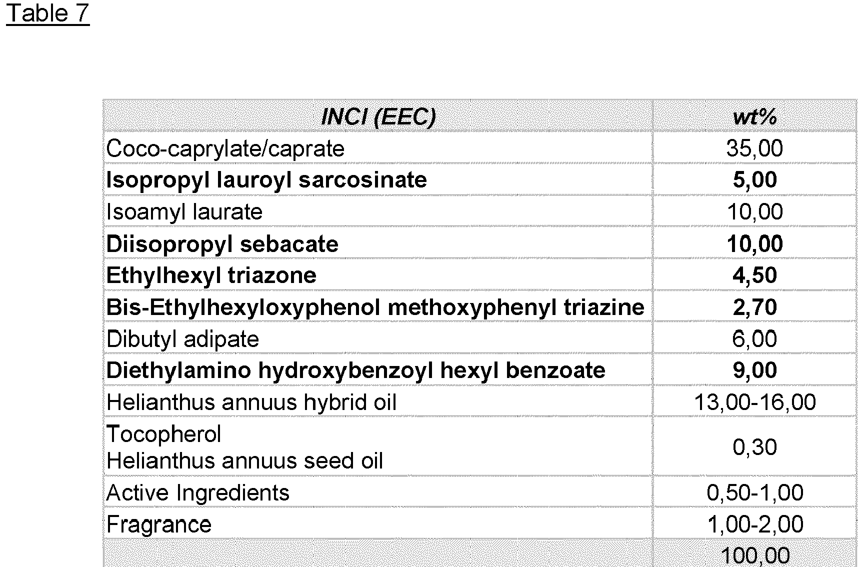

In bold : essential compounds of the invention
Calculated SPF: 34,2

### Manufacturing procedure:

Similar to example 4.

This ready to use oil preparation includes apart the esters mixture of the invention, other esters selected from coco caprylate/caprate in a high amount of 35 wt%, and isoamyl laurate. Compared to the oil sunscreen preparation of Example 4, the present oil preparation contains a higher amount of coco caprylate/caprate, since dextrin myristate is absent.

The ready to use sunscreen preparations of Examples 1-7 provide SPF *in vivo* values of higher that 10, and even of about 60-70 for some embodiments, with improved naturalness.

## Claims

1. A homogeneous sun protective topical composition comprising:
1) an effective amount of a UV filter system providing a Sun Protection Factor of 10 to 75, comprising a mixture of following compounds:
a) Diethylamino Hydroxybenzoyl Hexyl Benzoate (DHHB),
b) Ethylhexyl Triazone (EHT), and
c) Bis-ethylhexyloxyphenol Methoxyphenyl Triazine (BEMT), and
2) a mixture of ester compounds comprising:
a) Isopropyl lauroyl sarcosinate, present in a relative weight% range of from 2 to 50, and
b) Diisopropyl sebacate, present in a relative wt% range of from 5 to 65.

2. The homogeneous sun protective topical composition according to claim 1, **characterized in that** the UV filter system is the mixture wherein the relative weight%, based on 100% weight of the UV filter system, for DHHB is of from 35 to 60, for EHT is of from 10 to 35, and for BEMT of from 5 to 30, independently each other.

3. The homogeneous sun protective topical composition according to claim 2, **characterized in that**, independently each other, the DHHB relative weight% is of from 40 to 60, EHT weight% is of from 15 to 30, and BEMT weight% is of from 7 to 27.

4. The homogeneous sun protective topical composition according to any of claims 1 to 3, **characterized in that** the relative weight% between the UV filter system and the mixture of ester compounds is of from 0,10 to 1,5.

5. The homogeneous sun protective topical composition according to any of claims 1 to 4, **characterized in that** the UV filter system comprises of from 0 to 25 relative wt%, based on 100 wt% of the UV filter system, phenyl benzimidazole sulfonic acid (PBSA).

6. The homogeneous sun protective topical composition according to any of claims 1 to 5, **characterized in that** isopropyl lauroyl sarcosinate is present in a relative weight% range of from 7 to 45.

7. The homogeneous sun protective topical composition according to any of claims 1 to 6, **characterized in that** diisopropyl sebacate is present in a relative wt% range of from 10 to 60.

8. The homogeneous sun protective topical composition according to any of claims 1 to 6, **characterized in that** it comprises of from 0 to 70 relative wt% of dibutyl adipate.

9. The homogeneous sun protective topical composition according to any of claims 1 to 8, **characterized in that** the mixture of ester compounds comprises at least one additional ester selected from the group consisting in, based on 100 wt% of the esters mixture, of from 0 to 35, more preferably of from 0 to 30 relative wt% of C₁₂-C₁₅ alkylbenzoate, of from 0 to 60, more preferably of from 0 to 55 relative wt% of coco caprylate/caprate, of from 0 to 20, more preferably of from 15 to 20 relative wt% of isoamyl laurate and of from 0 to 10, more preferably of from 0 to 5 relative wt% of dextrin myristate.

10. The homogeneous sun protection topical composition according to any of claims 1 to 9, **characterized in that** said composition devoid or substantially free of compounds selected from the group consisting of siloxanes and derivatives thereof; octocrylene; ethyhexyl methoxycinnamate; homosalate; and ethylhexylsalicylate, and mixtures thereof.

11. The homogeneous sun protective topical composition according to any of claims 1 to 10, **characterized in that** the UV filter system is providing a SPF value of from 30 to 75, more preferably of from 35 to 75.

12. A ready to use sunscreen preparation comprising the homogeneous sun protective topical composition according to any of claims 1 to 11.

13. Ready to use sunscreen preparation according to claim 12, wherein the homogeneous sun protective topical composition content is of from 25 wt% to 95 wt%, preferably of from 25 wt% to 85 wt%, based of 100 wt% of said preparation.

14. Ready to use sunscreen preparation according to claim 12 or 13, wherein when said preparation includes oil phase, the composition content is of from 70 wt% to 85 wt% and, independently, the relative wt% of dibutyl adipate is of from 8 to 18.

15. Ready to use sunscreen preparation according to claim 12 or 13, wherein, when said preparation are sprays, creams, lotions, foams, gels or milks, the weight% ratio between the UV filter system and the mixture of ester compounds is of from 0,5 to 1,5 and, independently, the relative wt% of dibutyl adipate is of from 15 to 70, even more better of from 18 to 65.
